# NEUE EUROPÄISCHE PATENTSCHRIFT

(11) **EP 0 249 680 B2**
(45) Veröffentlichungstag und Bekanntmachung des Hinweises auf die Entscheidung über den Einspruch: **20.03.2002**
(45) Hinweis auf die Patenterteilung: 28.07.1993
(21) Anmeldenummer: 87102950.0
(22) Anmeldetag: 02.03.1987
(51) Int. Cl.: A61N 1/36

(54) **Sensoranordnung zur Regelung implantierbarer Körperersatzteile**
Detection device for controlling a replaced body part
Dispositif de détection pour commander un élément remplaçant une partie du corps

(30) Priorität: 16.06.1986 DE 3620277
(43) Veröffentlichungstag der Anmeldung: 23.12.1987
(73) Patentinhaber: St. Jude Medical AB, 175 84 Järfälla (SE)
(72) Erfinder: Heinze, Roland, Dipl.-Ing., D-8000 München 80 (DE); Liess, Hans-Dieter, Prof. Dr. Ing., D-8193 Münsing (DE)
(74) Vertreter: Harrison, Michael Charles

(56) Entgegenhaltungen:
- EP-A- 0 147 820
- EP-A- 0 211 700
- DE-A- 3 152 963
- DE-A1- 2 929 498
- US-A- 3 638 640
- US-A- 4 266 554
- US-A- 4 463 341
- P.Horowitz and W.Hill, "The Art of Electronics" Cambridge University Press, 1984, seite 246
- Siliconix "Analog Switch Data Book", 1976 seite 1-152 (Figur "Industrial Control Multiplexing") sowie seite 1-153 (Figur "An 8 Channel Mux/Demux System")
- T.R. Fryer, "A Multichannel EEG Telemetry System Utilizing a PCM Subcarrier", Biotelemetry I, 202 (1974)
- M.L. Hull, C. D. Mote, "Precision PCM Telemetry in Ski Injury Research", Biotelemetry II, Basel 1974

## Beschreibung

Die Erfindung betrifft eine Sensoranordnung zur Regelung implantierbarer Körperersatzteile, wobei die Sensoranordnung über einen mindestens zweipoligen Katheter mit einer Auswerteschaltung verbunden ist.

Eine derartige Sensoranordnung für einen Herzschrittmacher ist aus der DE-PS 31 52 963 bekannt. Dabei wird mit einem in einer Herzkammer angeordneten Sensor, der über einen zweipoligen Katheter mit dem Herzschrittmacher verbunden ist, die Blutsauerstoffsättigung erfaßt. In manchen Fällen wäre die Erfassung mehrerer Meßparameter wünschenswert. Im Falle von Herzschrittmachern geben beispielsweise die Blutsauerstoffsättigung, die Bluttemperatur, der Blutdruck und der Leitwert des Blutes Aufschlüsse über die Frequenz, mit der der Herzschrittmacher arbeiten soll. Alle diese Sensoren können nicht im Herzschrittmacher selbst untergebracht werden, so daß die Meßsignale über den Katheter übertragen werden müssen. Der Katheter soll jedoch aus Gründen der Flexibilität und Zuverlässigkeit möglichst wenig Leitungen aufweisen.

Aus der Druckschrift "Siliconix, "Analog Switch Data Book", 1976, Seite 1-152 (Figur "Industrial Control Multiplexing") sowie Seite 1-153 (Figur "An 8 Channel Mux/Demux System")" ist eine Anordnung zur Übertragung von Daten bekannt, bei der auf einer Sendeseite mehrere Eingänge einem Multiplexer zugeführt werden und ein Signal über eine Datenübertragungsleitung einem Demultiplexer auf der Empfängerseite zugeführt wird. Ferner ist eine Synchronisationsleitung zwischen Multiplexer und Demultiplexer vorgesehen. Sender- und Empfängerseite weisen jeweils getrennte Stromversorgungen auf. Eine derartige Anordnung ist für eine Sensoranordnung der oben genannten Art ungeeignet, da eine zusätzliche Stromversorgung für die Sensoren beim Einsatz im Körper höchst problematisch ist.

Aufgabe der Erfindung ist es, mehrere Meßsignale mit einer Anordnung von mehreren Sensoren so zu übertragen, daß der Katheter möglichst wenig Leitungen benötigt und für die Sensoranordnung keine gesonderte Stromversorgung nötig ist.

Diese Aufgabe wird erfindungsgemäß durch die Merkmale des Anspruches 1 gelöst.

Durch den zeitlichen Versatz können die verschiedenen Meßsignale voneinander getrennt werden, ohne daß jeweils eine gesonderte Leitung des Katheters erforderlich ist.

Die zeitlich versetzte Aktivierung der Sensoren kann auf einfache Weise dadurch erreicht werden, daß die Sensoren über Schalter an die Leitungen des Katheters angeschlossen sind und daß die Schalter von der Steuerschaltung angesteuert werden.

In einer Ausführungsform steuert die Auswerteschaltung durch Codesignale für den Meßsignalursprung über die Steuerschaltung die Aktivierung der Sensoren. Die Aktivierung der Sensoren wird dabei zweckmäßigerweise von einem Decoder gesteuert, der von der Auswerteschaltung codierte Triggersignale als Codesignale über die Leitungen des Katheters empfängt. Der Decoder decodiert die von der Auswerteschaltung kommenden codierten Triggersignale und steuert direkt oder über eine zusätzliche Funktionsstufe - etwa einen Zähler - die Schalter an. Die Steuerschaltung ist dabei vorteilhafterweise so aufgebaut, daß sie keine eigene Zuleitung zur Auswerteschaltung benötigt, was den Einsatz eines Dreipolkatheters erfordern würde. Sie wird zweipolig parallel zu den Sensoren geschaltet, ohne deren Funktion zu beeinflußen.

In einer alternativen Ausführungsform steuert die Steuerschaltung selbsttätig die Aktivierung der Sensoren, wobei die entstehenden Meßsignale mit für den angesteuerten Sensor spezifischen Codesignalen versehen sind.

Dabei können die Sensoren in einfacher Weise von einer in der Steuerschaltung enthaltenen Timer-Schaltung gesteuert werden.

Die Codesignale können Puls-Phasen-moduliert sein. Dies kann dadurch erzielt werden, daß bei jeder Meßperiode eine Null-Marke durch ein außerhalb des Meßbereiches liegendes Signal gesetzt wird und daß die Codierung durch den Abstand des codierten Signals von dieser Nullmarke erreicht wird.

Die Codierung kann alternativ auch durch Amplituden-, Pulsdauer- oder Pulscodemodulation erfolgen.

Jedem Sensor kann ein gemeinsamer Signalwandler mit Speicher zugeordnet sein, wobei der Speicher bei Aktivierung eines Sensors dessen Meßwert abspeichert und der abgespeicherte Meßwert nach der Meßphase vom Signalwandler in einer für die Auswerteschaltung geeigneten Form codiert und an diese übertragen wird.

Diese Anordnung empfiehlt sich insbesondere bei Sensoren, bei denen Meß- und Sende'signal zeitlich getrennt sind.

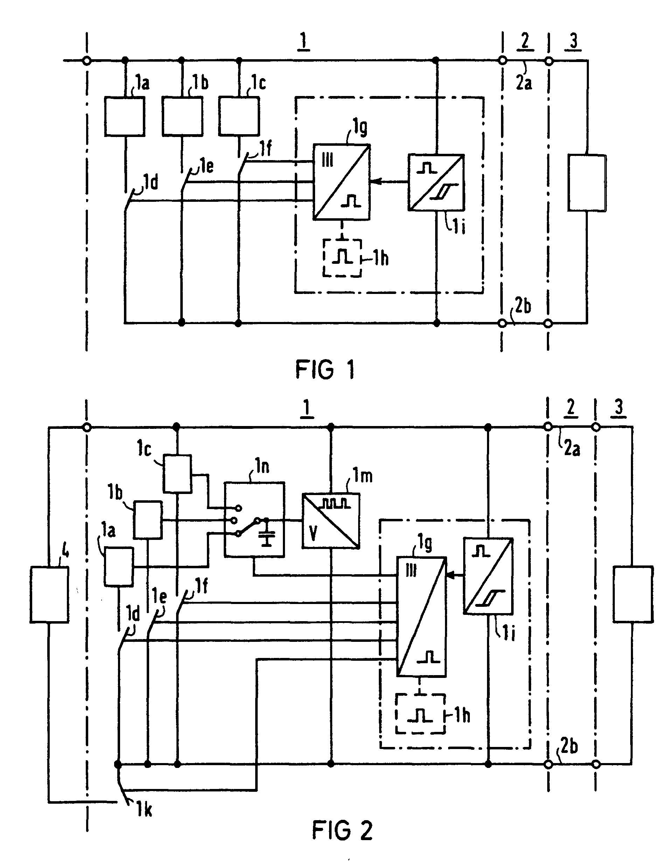

Ausführungsbeispiele der Erfindung werden nachfolgend anhand der Figuren 1 und 2 näher erläutert.

Figur 1 zeigt eine Sensoranordnung 1, die in einem Katheter 2 über zwei Leitungen 2a und 2b an eine Auswerteschaltung 3 angeschlossen ist. Die Sensoranordnung 1 mit den im Ausführungsbeispiel drei Sensoren 1a, 1b und 1c ist in einem gemeinsamen Sensorgehäuse untergebracht, das ebenfalls durch des Bezugszeichen 1 gekennzeichnet sein soll.

Die einzelnen Sensoren 1a bis 1c sind jeweils über Schalter 1d bis 1f an die Leitungen 2a und 2b des Katheters 2 angeschlossen. Die Schalter 1d bis 1f werden von einem Zähler 1g angesteuert. Der Zähler 1g kann entweder von einem der Sensoranordnung 1 eigenen Taktgeber 1h gesteuert werden oder über die Auswerteschaltung 3 und die Leitungen 2a und 2b durch einen Decoder 1i.

Die Leitungen 2a und 2b führen sowohl die Versorgungsströme für die gesamte Sensoranordnung 1 als auch die Meßströme. Die ist dann möglich, wenn die Versorgungsströme gegenüber den Meßströmen vernachlässigbar klein oder konstant sind, so daß die Meßströme durch Subtraktion der konstanten Versorgungsströme vom Gesamtstrom gewonnen werden können.

Die Schalter 1d bis 1f werden vom Zähler 1g zeitlich versetzt nacheinander eingeschaltet und damit der jeweils zugeordnete Sensor 1a bis 1c aktiviert, d.h. der eingeschaltete Sensor 1a bis 1c bestimmt den Meßwert (Spannung oder Strom) an den Leitungen 2a, 2b. Aufgrund eines Meßwertes oder der Kombination mehrerer dieser Meßwerte kann die Auswerteschaltung 3 eine Körperfunktion, beispielweise die Pulsfrequenz, regeln.

Einer der genannten Sensoren kann beispielsweise der in der bereits eingangs genannten DE-PS 31 52 963 erläuterte Sensor zur Erfassung der Blutsauerstoffsättigung sein.

Der Zähler 1g kann beispielsweise durch einen im Sensorgehäuse 1 angeordneten internen Takgeber 1h angesteuert werden, wobei der Zähler 1g und der Takgeber 1h zusammen als Timer-Schaltung wirken. In diesem Falle müssen die Meßsignale mit einer für den angesteuerten Sensor spezifischen Codierung versehen werden, damit die Auswerteschaltung 3 den Ursprung des Signals erkennt.

Alternativ kann die Auswerteschaltung 3 die Ansteuerung der Schalter 1d bis 1f und damit die Aktivierung der Sensoren 1a bis 1c steuern. In diesem Fall wird durch die Auswerteschaltung 3 über die Leitungen 2a, 2b ein Decoder 1i angesteuert der im Ausführungsbeispiel wiederum den Zähler 1g steuert. In diesem Fall müssen codierte Signale von der Auswerteschaltung 3 zum Decoder 1i übertragen werden.

Zur Codierung der von der Auswerteschaltung 3 an den Decoder 1i oder von den Sensoren 1a bis 1c an die Auswerteschaltung 3 zur Auswertung des Signalursprungs übertragenen Signale ist die Puls-Phasen-Modulation besonders günstig. Dabei wird bei jeder Meßperiode eine Nullmarke durch ein außerhalb des Meßbereichs liegendes Signal gesetzt. Mit dieser Nullmarke kann beispielsweise der Zähler 1g auf Null zurückgesetzt werden. Aus dem Abstand des empfangenen Signals zur Nullmarke kann dann bei bekannter Taktperiode geschlossen werden, welcher der Sensoren 1a bis 1c das jeweilige Signal abgegeben hat.

In einem Ausführungsbeispiel nach Fig. 2 ist den Sensoren 1a bis 1c ein gemeinsamer, an die Sensoren 1a bis 1c anschaltbarer Speicher 1n zugeordnet, an dessen Ausgang ein Signalwandler 1m angeschlossen ist. Der Signalwandler 1m ist wiederum an die Leitungen 2a, 2b angeschlossen. In diesem Fall sind Meß- und Sendesignal voneinander getrennt, wobei der Speicher 1n bei Aktivierung eines Sensors 1a bis 1c das gemessene Signal abspeichert und zeitlich nach der Meßphase der Signalwandler 1m den Meßwert in einer für die angeschlossene Auswerteschaltung 3 angepaßten Codierung an diese überträgt.

Als Codierung durch den Signalwandler kommen praktisch alle geläufigen Modulationsverfahren wie Amplituden-, Puls-Phasen-, Pulsdauer- und Pulscode-Modulation in Frage.

Beim Ausführungsbeispiel nach Figur 2 ist ein zusätzlicher Sensor 4 außerhalb des Sensorgehäuses untergebracht, der direkt an die Zuleitung 2a und über einen Schalter 1k an die Zuleitung 2b des Katheters 2 angeschlossen ist.

Der Schalter 1k wird über den Decoder 1i und über den Zähler 1g in analoger Weise wie die internen Sensoren 1a bis 1c gesteuert. Der Sensor 4 kann beispielsweise ein EKG-Sensor oder ein Impedanz-Sensor sein, die beide nicht innerhalb eines Sensorgehäuses untergebracht werden können.

## Patentansprüche

1. Sensoranordnung zur Regelung eines implantierbaren Körperersatzteiles mit folgenden Merkmalen:
- die Sensoranordnung (1) ist über einen mindestens zweipoligen, zwei elektrische Zuleitungen (2a, 2b) umfassenden Katheter (2) mit einer Auswerteschaltung (3) verbunden;
- in einem mit dem Katheter in Verbindung stehenden Sensorgehäuse sind mehrere Sensoren (1a bis 1c) untergebracht, die von einer ebenfalls im Sensorgehäuse (2) angeordneten gemeinsamen Steuerschaltung (1g bis 1i) zeitlich versetzt aktiviert werden,
- die entstehenden Meßsignale und Codesignale für den Meßsignalursprung werden Über die Zuleitungen (2a, 2b) des Katheters ebenfalls zeitlich versetzt übertragen;
- der Versorgungsstrom für die elektrischen Bauelemente im Sensorgshäuse wird ebenfalls über die Zuleitungen (2a, 2b) übertragen;
- der Versorgungsstrom ist konstant und/oder vernachlässigbar klein gegenüber Meßströmen.

2. Sensoranordnung nach Anspruch 1,
**dadurch gekennzeichnet,**
**daß** die Sensoren (1a bis 1c) Über Schalter (1d bis 1f) an die zuleitungen (2a, 2b) des Katheters (2) angeschlossen sind und daß die Schalter (1d bis 1f) von der Steuerschaltung (1g bis 1e) angesteuert werden.

3. Sensoranordnung nach Anspruch 1 oder 2.
**dadurch gekennzeichnet,**
**daß** die Auswerteschaltung (3) durch die Codesignale für den Meßsignalursprung über die Steuerschaltung (1g bis 1i) die Aktivierung der Sensoren (1d bis 1f) steuert.

4. Sensoranordnung nach Anspruch 3,
**dadurch gekennzeichnet,**
**daß** die Aktivierung der Sensoren (1d bis 1f) von einem Decoder (1i) gesteuert wird. der von der Auswerteschaltung (3) codierte Triggersignale als Codesignale über die zuleitungen (2a, 2b) des Katheters (2) empfängt.

5. Sensoranordnung nach Anspruch 1 oder 2,
**dadurch gekennzeichnet,**
**daß** die Steuerschaltung (1g, 1h) selbsttätig die Aktivierung der Sensoren (1a bis 1c) steuert und daß die entstehenden Meßsignale mit für den angesteuerten Sensor (1a bis 1c) spezifischen Codesignalen versehen sind.

6. Sensoranordnung nach Anspruch 5,
**dadurch gekennzeichnet,**
**daß** die Sensoren (1a bis 1c) von einer in der Steuerschaltung enthaltenen Timerschaltung (1h, 1g) gesteuert werden.

7. Sensoranordnung nach Anspruch 4 oder 5,
**dadurch gekennzeichnet**
**daß** die Codesignale Puls-Phasen-moduliert sind.

8. Sensoranordnung nach Anspruch 7,
**dadurch gekennzeichnet,**
**daß** die Puls-Phasen-Modulation dadurch erzielt wird, daß bei jeder Meßperiode eine Null-Marke durch ein außerhalb des Meßbereiches liegendes Signal gesetzt wird und daß die Codierung durch den Abstand des codierten Signals von dieser Null-Marke erreicht wird.

9. Sensoranordnung nach Anspruch 4 oder 5,
**dadurch gekennzeichnet,**
**daß** die Codierung durch Amplituden-, Pulsdauer- oder Pulscodernodulation erfolgt.

10. Sensoranordnung nach einem der Ansprüche 1 bis 9,
**dadurch gekennzeichnet,**
**daß** jedem Sensor (1a bis 1c) ein gemeinsamer Signalwandler (1m) mrt Speicher (1n) zugeordnet ist, daß der Speicher (1n) bei Aktivierung eines Sensors (1a bis 1c) dessen Meßwert abspeichert und daß der abgespeicherte Meßwert nach der Meßphase vom Signafwandler (1m) in einer für die Auswerteschattung (3) geeigneten Form codiert und an diese übertragen wird.

## Claims

1. Sensor arrangement for the control of an implantable body part replacement having the following features:
- the sensor arrangement (1) is connected to an evaluating circuit arrangement (3) by way of an at least two-pole catheter (2) which surrounds two electrical supply lines (2a, 2b);
- several sensors (1a to 1c) are accommodated in a sensor housing which communicates with the catheter, which sensors are activated in a temporally staggered manner by a common control circuit arrangement (1g to 1i) likewise arranged in the sensor housing (2) ;
- the resultant measured signals and code signals for the measured signal origin are transmitted by way of the electrical supply lines (2a, 2b) of the catheter likewise in a temporally staggered manner;
- the supply current for the electrical components in the sensor housing is transmitted likewise by way of the supply lines (2a, 2b);
- the supply current is constant and/or negligibly small compared with measured currents.

2. Sensor arrangement according to claim 1,
**chararcterised in that** the sensors (1a to 1c) are connected to the supply lines (2a, 2b) of the catheter (2) by way of switches (1d to 1f) and in that the switches (1d to 1f) are triggered by the control circuit arrangement (1g to 1e).

3. Sensor arrangement according to claim 1 or 2,
**characterised in that** the evaluating circuit arrangement (3) controls the activation of the sensors (1d to 1f) by means of the code signals for the measured signal origin by way of the control circuit arrangement (1g to 1i).

4. Sensor arrangement according to claim 3,
**characterised in that** the activation of the sensors (1d to 1f) is controlled by a decoder (1i) which receives trigger signals coded by the evaluating circuit arrangmeent (3) as code signals by way of the supply lines (2a, 2b) of the catheter (2).

5. Sensor arrangement according to claim 1 or 2,
**characterised in that** the control circuit arrangement (1g, 1h) automatically controls the activation of the sensors (1a to 1c) and **in that** the resultant measured signals are provided with specific code signals for the triggered sensor (1a to 1c).

6. Sensor arrangement according to claim 5,
**characterised in that** the sensors (1a to 1c) are controlled by a timer circuit arrangement (1h, 1f) contained in the control circuit arrangement.

7. Sensor arrangement according to claim 4 or 5,
**characterised in that** the code signals are pulse phase-modulated.

8. Sensor arrangement according to claim 7,
**characterised in that** the pulse phase-modulation is attained **in that** a zero mark is set for each measuring period by means of a signal which lies outside the measuring range and **in that** the coding is attained by the distance of the coded signal from this zero mark.

9. Sensor arrangement according to claim 4 or 5,
**characterised in that** the coding is effected by amplitude-modulation, pulse duration-modulation or pulse code-modulation.

10. Sensor arrangement according to one of the claims 1 to 9, **characterised in that** a common signal transducer (1m) with memory (1n) is associated with each sensor (1a to 1c), **in that** the memory (1n) in the case of activation of a sensor (1a to 1c) stores the measured value of the latter and **in that** the stored measured value after the measuring phase is coded by the signal transducer (1m) in a form suitable for the evaluating circuit arrangement (3) and is transmitted to the latter.

## Revendications

1. Dispositif formant détecteur pour régler un élément implantable suppléant une partie du corps, présentant les caractéristiques suivantes :
- le dispositif formant détecteur (1) est raccordé à un circuit d'exploitation (3) par l'intermédiaire d'au moins un cathéter bipolaire (2) comportant deux conducteurs (2a,2b) électriques d'alimentation;
- dans un boîtier du détecteur, qui est raccordé au cathéter, sont loges plusieurs capteurs (1a à 1c), qui sont activés, d'une manière décalée dans le temps, par un circuit de commands commun (1g à 1i) également disposé dans le boîtier (2) du détecteur,
- les signaux de mesure qui apparaissent et les signaux de code pour l'origine des signaux de mesure sont transmis, également d'une manière décalée dans le temps, au moyen des conducteurs d'alimentation (2a, 2b) du cathéter;
- le courant d'alimentation pour les composants électriques situés dans le boîtier du détecteur est également transmis par l'intermédiaire des conducteurs d'alimentation (2a, 2b);
- le courant d'alimentation est constant et/ou faible au point d'être négligeable par rapport à des courants de mesure.

2. Dispositif formant détecteur suivant la revendication 1, **caractérisé par le fait que** les capteurs (1a à 1c) sont raccordés, par l'intermédiaire d'interrupteurs (1d à 1f), aux conducteurs (2a, 2b) d'alimentation du cathéter (2) et que les interrupteurs (1d à 1f) sont commandés par le circuit de commande (1g à 1e).

3. Dispositif formant détecteur suivant la revendication 1 ou 2, **caractérisé par le fait que** le circuit d'exploitation (3) commande l'activation des capteurs (1d à 1f) à l'aide des signaux de code pour l'origine des signaux de mesure, par l'intermédiaire du circuit de commande (1g à 1i).

4. Dispositif formant détecteur suivant la revendication 3, **caractérisé par le fait que** l'activation des capteurs (1d à 1f) est commandées par un décodeur (1i) qui reçoit des signaux de déclenchement, qui sont codés par le circuit d'exploitation (3), en tant que signaux de code, par l'intermédiaire des conducteurs (2a,2b) d'alimentation du cathéter (2).

5. Dispositif formant détecteur suivant la revendication 1 ou 2, **caractérisé par le fait que** le circuit de commande (1g, 1f) commande automatiquement l'activation des capteurs (1a à 1c) et que les signaux de mesure, qui apparaissent, sont pourvus de signaux de code spécifiques au capteur commandé (1a à 1c).

6. Dispositif formant détecteur suivant la revendication 5, **caractérisé par le fait que** les capteurs (1a à 1c) sont commandés par un circuit formant minuterie (1h, 1g) présent dans le circuit de commande.

7. Dispositif formant détecteur suivant la revendication 4 ou 5, **caractérisé par le fait que** les signaux de code sont modulés selon une modulation d'impulsions en position.

8. Dispositif formant détecteur suivant la revendication 7, **caractérisé par le fait que** la modulation d'impulsions en position est obtenue grâce au fait que pendant chaque période de mesure, un repère de zéro est positionné par un signal situé à l'extérieur de la gamme de mesure et que le codage est obtenu au moyen de la distance entre le signal codé et ce repère de zéro.

9. Dispositif formant détecteur suivant la revendication 4 ou 5, **caractérisé par le fait que** le codage est réalisé au moyen d'une modulation d'amplitude, d'une modulation d'impulsions en durée ou d'une modulation par impulsions codées.

10. Dispositif formant détecteur suivant la revendication 1 à 9, **caractérisé par le fait qu'**à chaque capteur (1a à 1c) est associé un transformateur commun de signaux (1m) comportant une mémoire (1n), que, lors de l'activation d'un capteur (1a à 1c), la mémoire (1n) mémorise la valeur de mesure de ce capteur, et qu'après la phase de mesure, la valeur de mesure mémorisée est codée, par un transformateur de signaux (1m) sous une forme convenant pour le circuit d'exploitation (3) et est transmise à ce circuit.
